# DEMANDE DE BREVET EUROPEEN

(11) **EP 1 820 489 A1**
(43) Date de publication de la demande: **22.08.2007**
(21) Numéro de dépôt: 07300793.2
(22) Date de dépôt: 14.02.2007
(51) Int. Cl.: A61K 8/25, A61K 8/81, A61K 8/88, A61Q 1/04

(54) **Composition cosmétique comprenant une polyoléfine et des particules de silice pyrogénée**

(30) Priorité: 15.02.2006 FR 0650544
(71) Demandeur: L'ORÉAL, 75008 Paris (FR)
(72) Inventeur: Jacques, Véronique, 92340 Bourg la Reine (FR); Schwartz, Véronique, 75014 Paris (FR); Ilekti, Philippe, 94700 Maison-Alfort (FR)
(74) Mandataire: Le Coupanec, Pascale A.M.P.

(57) **Abrégé**

La présente invention concerne une composition cosmétique, de maquillage et/ou de soin des matières kératiniques, comprenant au moins une polyoléfine présentant un point de fusion inférieur ou égal à environ 40 °C et des particules de silice pyrogénée, ladite composition ne s'écoulant pas sous son propre poids et présentant une dureté inférieure ou égale à environ 30 g, mesurée à environ 20 °C.

## Description

La présente invention concerne des compositions cosmétiques, notamment destinées au maquillage et/ou au soin des matières kératiniques, en particulier de la peau et/ou des lèvres, comprenant au moins, au moins une polyoléfine et des particules de silice pyrogénée, ladite composition ne s'écoulant pas sous son propre poids et présentant une dureté inférieure ou égale à environ 30 g, mesurée à environ 20 °C.

Une composition selon l'invention peut, en particulier, être un produit de maquillage et/ou de soin destiné à être appliqué sur les matières kératiniques telles que la peau du corps, par exemple le visage et/ou les lèvres, ou les cils, et être notamment un gloss, un baume à lèvres, un fond de teint, notamment coulé en coupelle, un produit de coloration de la peau, un produit de maquillage des yeux comme un mascara ou un fard à joues ou à paupières, un produit anti-cerne ou un brillant à lèvres.

La présente invention a également pour objet un procédé de maquillage du visage et/ou du corps humain mettant en oeuvre une composition selon l'invention.

De nombreuses compositions cosmétiques existent pour lesquelles des propriétés de brillance et/ou d'effet coloré du film déposé sur la peau et/ou sur les lèvres sont recherchées. Ces propriétés participent généralement à l'effet esthétique désiré.

En particulier, des compositions comprenant une phase grasse gélifiée ou structurée peuvent être utilisées pour la préparation de compositions de maquillage des lèvres de type « gloss », qui selon la nature des huiles de phase grasse, peuvent donner un dépôt brillant sur les lèvres.

Toutefois, ces compositions peuvent présenter une dureté insuffisante et/ou être sous une forme liquide ou faiblement visqueuse. Une telle formulation nécessite une attention particulière de l'utilisatrice dans le stockage du récipient contenant la composition afin d'éviter de le renverser. Par ailleurs, ces compositions nécessitent également la mise en oeuvre de moyens d'applications adaptés.

Ainsi, ces formulations sont peu conviviales d'utilisations, et la présence d'accessoires supplémentaires peut augmenter leur coût de production.

Par ailleurs, de telles compositions peuvent également présenter une instabilité au cours du temps se traduisant par une séparation de phase ou synérèse.

Afin d'éviter ces inconvénients, ces compositions peuvent être structurées au moyen d'agents les rendant beaucoup plus dures mais se traduisant alors souvent par une altération de leur brillance initiale.

En conséquence, il existe un besoin de disposer de compositions cosmétiques qui possèdent une consistance suffisante pour ne pas couler sous son propre poids et, par exemple, compatible avec une application avec le doigt, tout en manifestant une fluidité propice à un étalement rapide et facile sur le support considéré, par exemple les lèvres.

Il existe également un besoin de disposer de compositions cosmétiques qui, en outre, n'induisent pas de sensation collante, après ou lors de leur application.

Il existe également un besoin de disposer de compositions cosmétiques qui soient stables au cours du temps, et qui ne soient pas altérées par des variations de température, notamment par exemple liées aux changements d'environnement extérieur/intérieur.

Il existe également un besoin de disposer de compositions qui présentent un aspect translucide et/ou transparent, et qui présentent une brillance améliorée lors de leur application.

La présente invention a pour objet de satisfaire à l'ensemble de ces besoins.

Ainsi, selon un de ses aspects, la présente invention a pour objet une composition cosmétique de maquillage et/ou de soin des matières kératiniques comprenant au moins une polyoléfine présentant un point de fusion inférieur ou égal à environ 40 °C, et des particules de silice pyrogénée, ladite composition ne s'écoulant pas sous son propre poids et présentant une dureté inférieure ou égale à environ 30 g, mesurée à environ 20 °C.

Selon un mode particulier de réalisation, une composition selon l'invention peut comprendre en outre au moins un polyamide de masse moléculaire moyenne inférieure à 100 000, comportant a) un squelette polymérique ayant des motifs de répétition hydrocarbonés pourvus d'au moins une fonction amide, b) optionnellement une chaîne grasse pendante et/ou au moins une chaîne grasse terminale, éventuellement fonctionnalisée, ayant de 6 à 120 atomes de carbones, et étant liée à ces motifs hydrocarbonés.

De manière inattendue, les inventeurs ont observé qu'il était possible de donner satisfaction à l'ensemble des exigences précitées sous réserve d'associer au sein d'une telle composition des particules de silice pyrogénée avec un polyisobutylène dans des conditions propices pour structurer celui-ci et conférer à la composition une dureté telle qu'elle est inférieure ou égale à environ 30 g, mesurée à environ 20 °C.

Ces compositions présentent également l'avantage d'être transparentes et/ou translucides et peuvent conférer une brillance améliorée au final, notamment sur les lèvres.

En particulier, du fait de cette translucidité, l'effet des matières colorantes, en particulier des nacres présentes dans les compositions est exalté dans le conditionnement.

Une composition conforme à l'invention peut être destinée à être appliquée sur les matières kératiniques, et notamment la peau du corps et/ou du visage, les lèvres, ou les phanères, tels que les cils.

En particulier, une composition selon l'invention peut être une composition pour les lèvres.

Elle peut se présenter sous la forme d'un gloss.

Elle peut également se présenter sous une forme compacte.

Ainsi, elle peut être appliquée au doigt ou à l'aide d'un applicateur adapté, tel que par exemple un embout mousse.

Selon un autre de ses aspects, la présente invention a pour objet une utilisation de particules de silice pyrogénée à titre d'agent de structuration d'une polyoléfine conforme à l'invention pour la préparation d'une composition cosmétique.

Selon un autre aspect, la présente invention a encore pour objet une utilisation d'une composition cosmétique conforme à l'invention pour l'obtention d'un dépôt brillant.

Selon encore un autre aspect, la présente invention a pour objet un procédé de maquillage et/ou de soin des matières kératiniques, et notamment de la peau et/ou des lèvres, comprenant au moins une étape consistant à appliquer sur au moins une partie desdites matières kératiniques d'un support une composition conforme à l'invention.

D'autres caractéristiques, aspects, objets et avantages de la présente invention apparaîtront encore plus clairement à la lecture de la description et des exemples qui suivent.

Selon un aspect, la phase grasse d'une composition selon l'invention, et en particulier une polyoléfine conforme à l'invention, est gélifiée ou structurée.

Par « phase grasse gélifiée » au sens de la présente invention, on entend désigner une phase grasse sous forme d'un gel, c'est-à-dire un réseau tridimensionnel qui retient dans ses mailles une quantité importante de solvant.

Par « phase grasse structurée », au sens de la présente demande, on entend désigner une phase grasse sous forme d'un gel rigide, présenté en coupelle, qui ne s'écoule pas sous son propre poids.

Une composition conforme à l'invention présentant une phase gélifiée ou structurée telle que la composition ne s'écoule pas sous son propre poids, à température ambiante, et qu'elle présente une dureté inférieure ou égale à environ 30 g, mesurée à environ 20 °C.

### MESURE DE LA DURETÉ

La dureté d'une composition selon l'invention peut être déterminée par une mesure de la force en compression, à 20 °C, à l'aide d'un texturomètre, par exemple vendu sous la dénomination TA-XT2i par la société RHEO, équipé d'un cylindre en inox d'un diamètre de 3 mm se déplaçant à une vitesse de mesure de 0,5 mm/s et pénétrant dans la composition cosmétique à une profondeur de pénétration de 2 mm.

Par exemple, le protocole de mesure peut être tel que défini ci-après.

Un échantillon de composition cosmétique est chauffé à une température de 90-100 °C. L'échantillon fondu est ensuite coulé dans un récipient de 4 mm de profondeur puis est refroidi à une température ambiante d'environ 20-25 °C pendant 24 heures.

L'échantillon est ensuite conservé pendant au moins une heure à 20 °C avant d'effectuer la mesure de dureté.

La valeur de dureté est la force de compression maximale mesurée, divisée par la surface du cylindre du texturomètre en contact avec l'échantillon de composition cosmétique.

Une composition cosmétique conforme à l'invention peut ainsi présenter une dureté variant de 6 g à environ 30 g, en particulier d'environ 10 g à environ 25 g, et plus particulièrement étant d'environ 15 g, mesurée à environ 20 °C.

Parallèlement à cette dureté, une composition selon l'invention peut être avantageusement dénuée de la faculté de s'écouler sous son propre poids.

Au sens de la présente invention, on entend désigner par « composition ne s'écoulant pas sous son propre poids », une composition présentant un comportement rhéologique à température ambiante tel que, lorsque après avoir été chauffée à une température de 90-100 °C, puis coulée dans un récipient par exemple de type cylindrique, d'un diamètre compris entre 2 et 3 cm, d'environ 2-3 cm de profondeur, puis refroidie à une température ambiante d'environ 20-25 °C pendant 24 heures, et que ce récipient est incliné de manière telle que l'axe passant par son orifice forme avec l'axe vertical un angle de 90 °C, pendant une durée d'environ 6 heures, la composition, remplissant au moins la moitié du récipient, ne s'écoule pas à l'extérieur du récipient.

La composition selon l'invention peut présenter un comportement rhéologique particulier, défini notamment par une viscosité pour une gamme de cisaillement définie et mesurée comme suit.

Les mesures peuvent être effectuées au moyen d'un rhéomètre à contrainte imposée, RS 75 de la société ThermoRhéo, équipé d'un bain thermostaté et d'un mobile en acier inoxydable à géométrie cône /plan, le cône ayant un diamètre de 35mm, un angle de 2° et un entrefer (distance entre le plan inférieur - appelé plan stator - sur lequel est déposée la composition et le plan supérieur - appelé plan rotor) de 0,3 mm.

Les mesures sont effectuées à 25°C ± 0,5°C.

L'analyse en régime d'écoulement à l'équilibre consiste à soumettre un échantillon de composition, à partir d'un instant donné, à une contrainte de cisaillement τ instantanée (cisaillement), maintenue constante pendant un temps t (temps d'attente choisi de façon à ce que le régime permanent soit atteint, t = 30s).

Simultanément, on suit l'évolution au cours du temps de la déformation de cisaillement correspondante γ et on enregistre le gradient de cisaillement 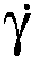 lorsque l'équilibre est atteint.

Dans un premier temps, l'échantillon est mis en température à 25°C pendant 2 min (sans aucun cisaillement appliqué).

Puis l'écoulement à l'équilibre est mesuré en mode contrainte imposée.

On applique des contraintes croissantes à l'échantillon en partant d'une contrainte initiale égale à 0.089 Pa pour arriver à une contrainte finale de 3400 Pa, les contraintes n'étant appliquées qu'une seule fois.

On attend l'obtention d'une valeur stable entre chaque contrainte, le temps d'attente entre chaque contrainte étant de 30 s.

La gamme de gradient de cisaillement va de 10⁻⁴ s⁻¹ à 10³ s⁻¹. Dans la gamme considérée, la valeur maximale de 10³ s⁻¹ doit être prise en compte avec une incertitude de mesure de ± 150 s⁻¹.

L'analyse des résultats se fait au travers de la représentation graphique de l'évolution de la viscosité, notée η, en fonction du gradient de cisaillement, noté 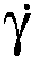 .

La composition selon l'invention peut présenter, avantageusement, un profil rhéologique tel que, pour une gamme de gradient de cisaillement allant 10⁻² à 10 s⁻¹, la viscosité est supérieure à 10⁵ mPas.

On entend désigner par « température ambiante » ci-dessus, la température de l'environnement dans lequel est disposée une composition et qui peut être fixée, pour des conditions expérimentales reproductibles, entre 20 et 25 °C, mais qui peut être amenée à varier, notamment en fonction des conditions saisonnières et de la nature de l'environnement.

### POLYOLEFINES

Une polyoléfine convenant à la mise en oeuvre de la présente invention présente un point de fusion inférieur ou égal à 40 °C.

En particulier, une polyoléfine convenant à la mise en oeuvre de l'invention peut être liquide à température ambiante (20-25 °C) et à pression atmosphérique.

Au regard des polyoléfines susceptibles d'être mises en oeuvre dans l'invention, l'expression « liquide à 20-25 °C » doit s'entendre comme visant à désigner des polyoléfines qui sont liquides au moins à cette température.

Au sens de l'invention, on entend par polyoléfine, une macromolécule obtenue par la polymérisation de motifs oléfiniques linéaires ou ramifiés de 3 à 20 atomes de carbone, en particulier en C₄ à C₁₆, en particulier en C₈ à C₁₂ et plus particulièrement en C₁₀ et comportant au moins une insaturation.

Elles peuvent être naturelles ou synthétiques

Les motifs oléfiniques peuvent être de type alphaoléfinique.

Selon un mode de réalisation, une polyoléfine susceptible de convenir à la mise en oeuvre de la présente invention peut être une polyalphaoléfine.

En particulier, les polyalphaoléfines convenant à la mise en oeuvre de la présente invention peuvent résulter de la polymérisation et co-polymérisation d'oléfines de faible poids moléculaire et présenter des chaînes fondamentales courtes par exemple en C₃ à C₁₀ et des chaînes latérales longues, par exemple en C₄ à C₁₂.

A titre d'exemple de motifs oléfiniques convenant à l'invention, on peut mentionner l'isobutylène, le décène.

En particulier, une polyoléfine convenant à l'invention peut être non volatile et peut présenter une pression de vapeur inférieure à 0,13 Pa.

Selon un mode de réalisation, une polyoléfine convenant à la mise en oeuvre de l'invention peut être de masse molaire élevée.

Par « polyoléfine liquide de masse molaire élevée », on entend désigner des polyoléfines liquides ayant une masse molaire inférieure ou égale à 10 000 g/mol, allant par exemple de 400 à environ 10 000 g/mol, en particulier de 600 à 7500 g/mol et plus particulièrement variant de 650 à 5000 g/mol.

Selon un mode de réalisation, une polyoléfine ou polyalphaoléfine pouvant convenir à l'invention peut être hydrogénée.

Selon un mode de réalisation, une polyoléfine convenant à l'invention peut être choisie parmi :
- des polybutylènes tels que l'INDOPOL H-100 (de masse molaire MM = 965 g/mol), l'INDOPOL H-300 (MM = 1340 g/mol), l'INDOPOL H-1500 (MM = 2160 g/mol), commercialisés ou fabriqués par la société AMOCO,
- des polyisobutylènes, hydrogénés ou non, tel que le PARLEAM de masse molaire faible ou élevée, commercialisé ou fabriqué par la société NOF CORPORATION, le PANALANE H-300 E commercialisé ou fabriqué par la société AMOCO (MM = 1340 g/mol), le VISEAL 20 000 commercialisé ou fabriqué par la société SYNTEAL (MM = 6000 g/mol), le REWOPAL PIB 1000 commercialisé ou fabriqué par la société WITCO (MM = 1000 g/mol),
- des polydécènes, hydrogénés ou non, tels que le PURESYN 10 (MM = 723 g/mol), le PURESYN 150 (MM = 9200 g/mol), commercialisés ou fabriqués par la société MOBIL CHEMICALS,
- et leurs mélanges.

Une polyoléfine peut être présente dans une composition selon l'invention en une teneur variant d'environ 5 à environ 90 %, en poids, en particulier d'environ 10 à environ 60 % en poids, et plus particulièrement d'environ 30 % à environ 40 % en poids par rapport au poids total de la composition.

### SILICE PYROGENEE

La composition selon l'invention comprend, en outre, avantageusement des particules de silice pyrogénée.

Les particules de silice pyrogénée convenant à la mise en oeuvre de l'invention peuvent être hydrophiles ou être traitées en surface afin d'être rendues hydrophobes.

Les silices pyrogénées hydrophiles peuvent être obtenues par hydrolyse à haute température d'un composé volatil du silicium dans une flamme oxhydrique, produisant une silice finement divisée. Les silices hydrophiles peuvent présenter un nombre important de groupements silanol à leur surface.

De telles silices hydrophiles sont par exemple commercialisées sous les dénominations "AEROSIL 130^{®}", "AEROSIL 200^{®}", "AEROSIL 255^{®}", "AEROSIL 300^{®}", "AEROSIL 380^{®}" par la société Degussa, "CAB-O-SIL HS-5^{®}", "CAB-O-SIL EH-5^{®}", "CAB-O-SIL LM-130^{®}", "CAB-O-SIL MS-55^{®}", "CAB-O-SIL M-5 par la société Cabot.

Les silices pyrogénées hydrophobes peuvent être obtenues par modification de la surface de la silice par une réaction chimique générant une diminution du nombre de groupes silanols, ces groupes pouvant être notamment substitués par des groupements hydrophobes.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6^{ème} édition, 1995). Elles sont par exemple commercialisées sous les références "AEROSIL R812^{®}" par la société Degussa, "CAB-O-SIL TS-530^{®}" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6^{ème} édition, 1995). Elles sont par exemple commercialisées sous les références "AEROSIL R972^{®}", "AEROSIL R974^{®}" par la société Degussa, "CAB-O-SIL TS-610^{®}", "CAB-O-SIL TS-720^{®}" par la société Cabot.

D'une manière générale, les particules de silice sont présentes en une quantité suffisante pour ajuster la dureté des compositions selon l'invention à la valeur requise.

Des particules de silice pyrogénée peuvent être présentes dans une composition conforme à l'invention en une teneur variant d'environ 1 % à environ 30 % en poids, en particulier d'environ 5 à environ 20 % en poids, et plus particulièrement d'environ 8 % à environ 15 % en poids par rapport au poids total de la composition.

Selon un mode de réalisation, la teneur de l'ensemble des composés entrant dans une composition conforme à l'invention, à savoir notamment au moins une polyoléfine selon l'invention, et des particules de silice pyrogénées, peut être ajustée à 100 %.

D'autres composés usuellement utilisés en cosmétique, tels que des phases grasses liquides ou solides, des actifs cosmétiques, des charges ou des matières colorantes, peuvent être additionnellement présents en des quantités ajustées de manière à ce que les propriétés d'une composition conforme à l'invention, notamment en terme de dureté, de brillance et/ou de transparence n'en soient pas substantiellement altérées.

### POLYAMIDE

Une composition selon la présente invention peut comprendre au moins un polymère de masse moléculaire moyenne en poids inférieure à 100 000, notamment inférieure à 50 000, comportant a) un squelette polymérique ayant des motifs de répétition hydrocarbonés pourvus d'au moins une fonction amide, et b) optionnellement au moins une chaîne grasse pendante et/ou au moins une chaîne grasse terminale éventuellement fonctionnalisées, ayant de 6 à 120 atomes de carbone, et notamment de 8 à 120 atomes de carbone, et étant liées à ces motifs hydrocarbonés.

Par "chaînes fonctionnalisées" au sens de l'invention, on entend une chaîne alkyle comportant un ou plusieurs groupes fonctionnels ou réactifs notamment choisis parmi les groupes amides, hydroxyle, éther, oxyalkylène ou polyoxyalkylène, halogène dont les groupes fluorés ou perfluorés, ester, siloxane, polysiloxane. En outre, les atomes d'hydrogène d'une ou plusieurs chaînes grasses peuvent être substitués au moins partiellement par des atomes de fluor.

Selon l'invention, ces chaînes peuvent être liées directement au squelette polymérique ou via une fonction ester ou un groupement perfluoré.

Par « polymère », on entend au sens de l'invention un composé ayant au moins 2 motifs de répétition, et de préférence au moins 3 motifs de répétition.

Par « motif de répétition hydrocarbonés », on entend au sens de l'invention un motif comportant de 2 à 80 atomes de carbone, et de préférence de 2 à 60 atomes de carbone, portant des atomes d'hydrogène et éventuellement des atomes d'oxygène, qui peut être linéaire, ramifié ou cyclique, saturé ou insaturé. Ces motifs comprennent, en outre, chacun d'une à plusieurs fonctions amide avantageusement non pendantes et se trouvant dans le squelette polymérique.

Selon un mode de réalisation, des chaînes grasses pendantes peuvent être liées directement à l'un au moins des atomes d'azote des motifs amide.

Un polyamide convenant à l'invention peut comprendre, entre les motifs hydrocarbonés, des motifs siliconés ou des motifs oxyalkylénés.

En outre, les chaînes grasses d'un polyamide convenant à la mise en oeuvre de la composition de l'invention peuvent présenter de 40 à 98 % du nombre total des motifs amide et des chaînes grasses, et en particulier de 50 à 95 % du nombre total des motifs amide et des chaînes grasses.

Avantageusement, un polyamide selon l'invention peut présenter une masse moléculaire moyenne en poids inférieure à 100 000, en particulier variant de 1000 à 100 000, en particulier variant de 1000 à 50 000, en particulier variant de 1000 à 30 000, en particulier variant de 2000 à 20 000, et plus particulièrement variant de 2000 à 10 000 g/mol.

Un polyamide convenant à l'invention peut être non soluble dans l'eau, notamment à 25 °C. En particulier, il ne comporte pas de groupe ionique.

A titre d'exemple de polyamides utilisables dans l'invention, on peut citer les polyamides ramifiés par des chaînes grasses pendantes et/ou des chaînes grasses terminales ayant de 6 à 120 atomes de carbone et mieux de 8 à 120 et notamment de 12 à 68 atomes de carbone.

En particulier, une ou les chaîne(s) grasse(s) terminale(s) peu(ven)t être liée(s) au squelette polyamide par des groupes de liaison, en particulier des groupes ester.

En particulier, un polyamide convenant à l'invention peut comporter une chaîne grasse à chaque extrémité du squelette polymérique. Comme autre groupe de liaison convenant, on peut citer les groupes éther, amine, urée, uréthane, thioester, thiurée, thiouréthane.

Un polyamide convenant à l'invention peut être un polyamide, résultant d'une polycondensation d'un diacide carboxylique ayant au moins 32 atomes de carbone (ayant notamment de 32 à 44 atomes de carbone) avec une amine choisie parmi les diamines ayant au moins 2 atomes de carbone (notamment de 2 à 36 atomes de carbone) et les triamines ayant au moins 2 atomes de carbone (notamment de 2 à 36 atomes de carbone).

Un diacide convenant à l'invention peut être par exemple un dimère issu d'acide gras à insaturation éthylénique ayant au moins 16 atomes de carbone, en particulier de 16 à 24 atomes de carbone, comme l'acide oléique, linoléique ou linolénique.

A titre d'exemple de diamine, on peut mentionner l'éthylène diamine, l'hexylène diamine, l'hexaméthylène diamine. La triamine peut être par exemple l'éthylène triamine.

Pour les polymères comportant un ou 2 groupements d'acide carboxylique terminaux, il peut être avantageux de les estérifier par un monoalcool ayant au moins 4 atomes de carbone, en particulier de 10 à 36 atomes de carbone et plus particulièrement de 12 à 24 et en particulier de 16 à 24, par exemple 18 atomes de carbone.

Ces polymères peuvent être par exemple ceux décrits dans le document US-A-5783657 de la société Union Camp.

Selon une variante de réalisation, le polyamide peut être de formule générale (I) suivante : 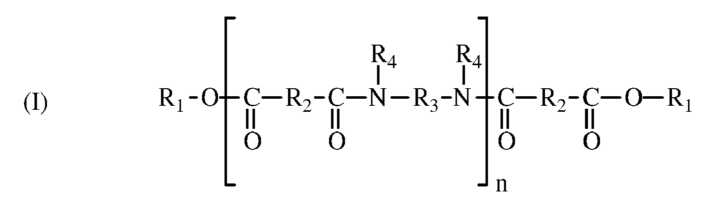 dans laquelle :
- n peut désigner un nombre entier de motifs amide tel que le nombre de groupes ester représente de 10 % à 50 % du nombre total des groupes ester et amide ;
- chacun des symboles R₁ peut désigner indépendamment un groupe alkyle ou alcényle ayant au moins 4 atomes de carbone et en particulier de 4 à 24 atomes de carbone ;
- chacun des symboles R₂ peut représenter indépendamment un groupe hydrocarboné en C₄ à C₄₂, à condition que 50 % des groupes R₂ représentent un groupe hydrocarboné en C₃₀ à C₄₂ ;
- chacun des symboles R₃ peut représenter à chaque occurrence indépendamment un groupe organique pourvu d'au moins 2 atomes de carbone, d'atomes d'hydrogène et optionnellement d'un ou plusieurs atomes d'oxygène ou d'azote ; et
- chacun des symboles R₄ peut représenter indépendamment un atome d'hydrogène, un groupe alkyle en C₁ à C₁₀ ou une liaison directe à R₃ ou à un autre R₄ de sorte que l'atome d'azote auquel sont liés à la fois R₃ et R₄ fasse partie d'une structure hétérocyclique définie par R₄-N-R₃, avec au moins 50 % des R₄ représentant un atome d'hydrogène.

Dans le cas particulier de la formule (I), les chaînes grasses terminales éventuellement fonctionnalisées au sens de l'invention sont des chaînes terminales liées au dernier atome d'azote du squelette polyamide.

En particulier, les groupes ester de la formule (I), qui font partie des chaînes grasses terminales et/ou pendantes au sens de l'invention, peuvent représenter de 15 à 40 % du nombre total des groupes ester et amide et en particulier de 20 à 35 %.

De plus, n peut représenter avantageusement un nombre entier allant de 1 à 5 et en particulier supérieur à 2.

R₁ peut être un groupe alkyle en C₁₂ à C₂₂ et en particulier en C₁₆ à C₂₂.

R₂ peut être un groupe hydrocarboné (alkylène) en C₁₀ à C₄₂, et en particulier, 50 % au moins et plus particulièrement au moins 75 % des R₂ peuvent être des groupes hydrocarbonés ayant de 30 à 42 atomes de carbone. Les autres R₂ peuvent être des groupes hydrocarbonés en C₄ à C₁₉ et en particulier en C₈ à C₁₂.

R₃ peut représenter un groupe hydrocarboné en C₂ à C₃₆ ou un groupe polyoxyalkyléné.

En particulier, R₃ peut représenter un groupe hydrocarboné en C₄ à C₁₂.

R₄ peut représenter un atome d'hydrogène.

Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés, saturés ou non.

Les polymères de formule (I) peuvent se présenter sous forme de mélanges de polymères. Ces mélanges peuvent en outre contenir un produit de synthèse correspondant à un composé de formule (I) où n vaut 0, c'est-à-dire un diester.

Selon un mode de réalisation, un polyamide convenant à l'invention peut être choisi parmi les copolymères de diacide en C₃₆ condensés sur une éthylène diamine de masse moléculaire en poids d'environ 6 000, et leurs mélanges.

A titre d'exemple de polymères utilisables dans les compositions selon l'invention, on peut citer les produits commerciaux vendus par la société Arizona Chemical sous les noms Uniclear 80 et Uniclear 100. Ils sont vendus respectivement sous forme de gel à 80 % (en matière active) dans une huile minérale et à 100 % (en matière active). Ils ont un point de ramollissement de 88 à 94 °C. Ces produits commerciaux sont un mélange de copolymères d'un diacide en C₃₆ condensé sur l'éthylène diamine, de masse moléculaire moyenne en poids d'environ 6000. Les groupes ester terminaux résultent de l'estérification des terminaisons d'acide restantes par l'alcool cétylique, stéarylique ou leurs mélanges (appelés aussi alcool cétylstéarylique).

Comme polyamide utilisable dans les compositions selon l'invention, on peut encore citer les résines polyamides résultant de la condensation d'un acide di-carboxylique aliphatique et d'une diamine (incluant les composés ayant plus de 2 groupes carbonyle et 2 groupes amine), les groupes carbonyle et amine de motifs unitaires adjacents étant condensés par une liaison amide. Ces résines polyamides sont notamment celles commercialisées sous la marque Versamid^{®} par les sociétés Général Mills, Inc. et Henkel Corp. (Versamid 930, 744 ou 1655) ou par la société Olin Mathieson Chemical Corp., sous la marque Onamid^{®} notamment Onamid S ou C. Ces résines ont une masse moléculaire moyenne en poids allant de 6000 à 9000. Pour plus d'information sur ces polyamides, on peut se référer aux documents US-A-3645705 et US-A-3148125. Plus spécialement, on utilise les Versamid^{®} 930 ou 744.

On peut aussi utiliser les polyamides vendus par la société Arizona Chemical sous les références Uni-Rez (2658, 2931, 2970, 2621, 2613, 2624, 2665, 1554, 2623, 2662) et le produit vendu sous la référence Macromelt 6212 par la société Henkel. Pour plus d'information sur ces polyamides, on peut se référer au document US-A-5500209.

Il est aussi possible d'utiliser des résines de polyamides issues de légumes comme celles décrites dans les brevets US-A-5783657 et US-A-5998570.

Un polyamide convenant à la préparation d'une composition selon l'invention peut présenter une température de ramollissement supérieure à 65°C et pouvant aller jusqu'à 190 °C. En particulier, il peut présenter une température de ramollissement variant de 70 à 130 °C, et en particulier de 80 à 105 °C.

Un polyamide peut être présent dans une composition selon l'invention en une teneur variant d'environ 0,01 % à environ 20 % en poids, en particulier d'environ 0,1 % à environ 10 % en poids, et plus particulièrement d'environ 0,2 à environ 4 % en poids par rapport au poids total de la composition.

### MILIEU PHYSIOLOGIQUEMENT ACCEPTABLE

Une composition conforme à l'invention comprend un milieu physiologiquement acceptable.

Par « milieu physiologiquement acceptable », on entend désigner un milieu convenant particulièrement à l'application d'une composition selon l'invention sur la peau et/ou les lèvres. Le milieu physiologiquement acceptable est généralement adapté à la nature du support sur lequel doit être appliquée la composition, ainsi qu'à l'aspect sous lequel la composition est destinée à être conditionnée.

Le milieu physiologiquement acceptable peut comprendre une phase aqueuse comprenant, essentiellement d'eau.

Elle peut également comprendre un mélange d'eau et de solvant miscible à l'eau (miscibilité dans l'eau supérieure à 50 % en poids à 25 °C) comme un, ou un mélange de monoalcool(s) inférieur(s) ayant de 1 à 5 atomes de carbone tel(s) que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, les aldéhydes en C₂-C₄ et leurs mélanges.

Selon un autre mode de réalisation, la composition conforme à l'invention peut être anhydre.

Par composition anhydre, on entend une composition comprenant moins de 10 % en poids, en particulier moins de 5 % en poids, en particulier moins de 3 % en poids, en particulier moins de 2 % en poids, et plus particulièrement moins de 1 % en poids d'eau par rapport au poids total de la composition.

### PHASE GRASSE

Une composition cosmétique conforme à la présente invention peut comprendre, outre une polyoléfine conforme à l'invention, une phase grasse additionnelle, notamment une phase grasse liquide, comprenant une ou plusieurs huiles, et/ou une phase grasse solide à température d'environ 20 - 25 °C et pression atmosphérique, et leur mélange.

On entend par huile, tout corps gras sous forme liquide à température d'environ 20 - 25 °C et à pression atmosphérique. La phase grasse liquide peut, également, contenir outre des huiles, d'autres composés solubilisés dans les huiles tels que des agents gélifiants et/ou structurants.

La ou les huiles peuvent être présentes à raison de 0,1 à 99 % en poids, en particulier d'au moins 1 à 90 % en poids, plus particulièrement de 5 à 70 % en poids, notamment de 10 à 60 % en poids, voire de 20 à 50 % en poids, par rapport au poids total de la composition cosmétique selon l'invention.

La phase grasse liquide pouvant convenir à la préparation d'une composition cosmétique selon l'invention peut être choisie parmi des huiles volatiles ou non, siliconées ou non, et leurs mélanges.

Au sens de la présente invention, on entend par « huile siliconée », une huile comprenant au moins un atome de silicium, et notamment au moins un groupe Si-O.

On entend par « huile hydrocarbonée », une huile contenant principalement des atomes d'hydrogène et de carbone et éventuellement des atomes d'oxygène, d'azote, de soufre et/ou de phosphore.

### Huiles volatiles

Une composition conforme à l'invention peut comprendre au moins une huile volatile.

Au sens de la présente invention, on entend par « huile volatile », une huile (ou milieu non aqueux) susceptible de s'évaporer au contact de la peau en moins d'une heure, à température ambiante et à pression atmosphérique. L'huile volatile est une huile cosmétique volatile, liquide à température ambiante, ayant notamment une pression de vapeur non nulle, à température ambiante (20-25 °C) et pression atmosphérique, en particulier ayant une pression de vapeur allant de 0,13 Pa à 40 000 Pa (10⁻³ à 300 mm Hg), en particulier allant de 1,3 Pa à 13 000 Pa (0,01 à 100 mm Hg), et plus particulièrement allant de 1,3 Pa à 1300 Pa (0,01 à 10 mm Hg).

Les huiles hydrocarbonées volatiles peuvent être choisies parmi les huiles hydrocarbonées ayant de 8 à 16 atomes de carbone, et notamment les alcanes ramifiés en C₈-C₁₆ (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars^{®} ou de Permethyls^{®}.

Comme huiles volatiles, on peut aussi utiliser les huiles de silicones volatiles, comme par exemple les huiles de silicones linéaires ou cycliques volatiles, notamment celles ayant une viscosité ≤ 8 centistokes (8 x 10⁻⁶ m²/s), et ayant notamment de 2 à 10 atomes de silicium, et en particulier de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment les diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

On peut également utiliser des huiles volatiles fluorées telles que le nonafluorométhoxybutane ou le perfluorométhylcyclopentane, et leurs mélanges.

Il est également possible d'utiliser un mélange des huiles précédemment citées.

Une composition cosmétique selon l'invention peut comprendre au moins une huile volatile en une teneur variant d'environ 1 % à environ 50 % en poids, en particulier variant d'environ 2 % à environ 40 % en poids, et plus particulièrement variant d'environ de 5 % à environ 30 % en poids d'huile volatile par rapport au poids total de la composition.

### Huiles non volatiles

Au sens de la présente invention, on entend par « huile non-volatile », une huile ayant une pression de vapeur inférieure à 0,13 Pa. Les huiles non volatiles peuvent être des huiles hydrocarbonées notamment d'origine animale ou d'origine végétale, des huiles d'origine synthétique ou minérale, des huiles siliconées, des huiles fluorées, ou leurs mélanges.

Une composition cosmétique selon la présente invention peut ainsi comprendre en outre au moins une huile non volatile.

Les huiles non volatiles peuvent notamment être choisies parmi les huiles hydrocarbonées le cas échéant fluorées et/ou les huiles siliconées non volatiles.

Comme huile hydrocarbonée non volatile convenant à la mise en oeuvre de l'invention, on peut notamment citer :
- les huiles hydrocarbonées d'origine animale,
- les huiles hydrocarbonées d'origine végétale telles que les esters de phytostéaryle, tels que l'oléate de phytostéaryle, l'isostéarate de physostéaryle et le glutanate de lauroyl/octyldodécyle/phytostéaryle, par exemple vendu sous la dénomination ELDEW PS203 par AJINOMOTO, les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment des triglycérides héptanoïques ou octanoïques, les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de courge, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société STÉARINERIES DUBOIS ou ceux vendus sous les dénominations MIGLYOL 810^{®}, 812^{®} et 818^{®} par la société DYNAMIT NOBEL,
- les huiles hydrocarbonées d'origine minérale ou synthétique comme par exemple :
   - les éthers de synthèse ayant de 10 à 40 atomes de carbone ;
   - les hydrocarbures linéaires ou ramifiés, d'origine minérale ou synthétique tels que la vaseline liquide, le squalane et leurs mélanges,
   - les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10.

Les esters peuvent être notamment choisis parmi les esters, notamment d'acide gras comme par exemple :
- l'octanoate de cétostéaryle, les esters de l'alcool isopropylique, tels que le myristate d'isopropyle, le palmitate d'isopropyle, le palmitate d'éthyle, le palmitate de 2-éthyl-hexyle, le stéarate ou l'isostéarate d'isopropyle, l'isostéarate d'isostéaryle, le stéarate d'octyle, les esters hydroxylés comme le lactacte d'isostéaryle, l'hydroxystéarate d'octyle, l'adipate de diisopropyle, les heptanoates, et notamment l'heptanoate d'isostéaryle, octanoates, décanoates ou ricinoléates d'alcools ou de polyalcools comme le dioctanoate de propylène glycol, l'octanoate de cétyle, l'octanoate de tridécyle, le 4-diheptanoate et le palmitate d'éthyle 2-hexyle, le benzoate d'alkyle, le diheptanoate de polyéthylène glycol, le diétyl 2-d'hexanoate de propylèneglycol et leurs mélanges, les benzoates d'alcools en C₁₂ à C₁₅, le laurate d'hexyle, les esters de l'acide néopentanoïque comme le néopentanoate d'isodécyle, le néopentanoate d'isotridécyle, le néopentanoate d'isostéaryle, le néopentanoate d'octyldocécyle, les esters de l'acide isononanoïque comme l'isononanoate d'isononyle, l'isononanoate d'isotridécyle, l'isononanoate d'octyle, les esters hydroxylés comme le lactate d'isostéaryle, le malate de di-isostéaryle ;
   - les esters de polyols, et les esters de pentaétrythritol, comme le tétrahydroxystéarate/tétraisostéarate de dipentaérythritol,
   - les esters de dimères diols et dimères diacides tels que les Lusplan DD-DA5^{®} et Lusplan DD-DA7^{®}, commercialisés par la société NIPPON FINE CHEMICAL et décrits dans la demande FR 03 02809,
   - les alcools gras liquides à température ambiante à chaîne carbonée ramifiée et/ou insaturée ayant de 12 à 26 atomes de carbone comme le 2-octyldodécanol, l'alcool isostéarylique, l'alcool oléique, le 2-hexyldécanol, le 2-butyloctanol, et le 2-undécylpentadécanol,
   - les acides gras supérieurs tels que l'acide oléique, l'acide linoléique, l'acide linolénique et leurs mélanges, et
   - les carbonates de di-alkyle, les 2 chaînes alkyles pouvant être identiques ou différentes, tel que le dicaprylyl carbonate commercialisé sous la dénomination Cetiol CC^{®,} par Cognis,
   - les huiles de silicone non volatiles, comme par exemple les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates, les diméthicones ou phényltriméthicone de viscosité inférieure ou égale à 100 Cst, et leurs mélanges,
- et leurs mélanges.

Les huiles non volatiles peuvent être présentes dans une composition selon l'invention en une teneur allant de 5 à 90 % en poids, notamment de 25 % à 80 % en poids, et en particulier de 40 % à 70 % en poids, par rapport au poids total de la composition.

### Autres corps gras

Une composition selon l'invention peut également comprendre au moins une phase grasse solide choisie parmi les cires, les corps gras pâteux, et leurs mélanges.

La cire est solide à température ambiante (25 °C), à changement d'état solide/liquide réversible, ayant une température de fusion supérieure à 30 °C pouvant aller jusqu'à 200 °C, une dureté supérieure à 0,5 MPa et présentant à l'état solide une organisation cristalline anisotrope.

Elle peut être hydrocarbonée, fluorée et/ou siliconée et être d'origine animale, végétale, minérale ou synthétique.

Elle peut être choisie par exemple parmi la cire d'abeille, la cire de Carnauba, la cire de Candelilla, les cires de paraffine, l'huile de ricin hydrogénée, les cires synthétiques comme les cires de polyéthylène (de préférence de poids moléculaire compris entre 400 et 600) ou de Fischer-Tropsch, les cires de silicone comme les alkyl- ou alkoxy-diméthicone ayant de 16 à 45 atomes de carbone, les cérésines ou les ozokérites, comme par exemple les isoparaffines dont le point de fusion est inférieur à 40 °C, tel que « l'EMW-0003 », commercialisé par la société NIPPON SEIROU, les oligomères d'α-oléfine, tel que les polymères «PERFORMA V^{®} 825 », « 103 » et « 260 », commercialisés par la société NEW PHASE TECHNOLOGIES ; les copolymères éthylène-propylène, tel que le « PERFORMALENE^{®} EP 700 », et les cires microcristallines dont le point de fusion est supérieur à 85 °C, tel que les « HI-MIC^{®} 1070 », « 1080 », « 1090 » et « 3080 », commercialisées par NIPPON SEIROU, et leurs mélanges.

Selon un mode de réalisation, la teneur de la ou des cire(s) utilisée(s) dans une composition cosmétique conforme à l'invention peut être inférieure ou égale à environ 5 % en poids par rapport au poids total de la composition.

Selon un autre mode de réalisation, une composition conforme à l'invention peut être exempte de cire.

Une composition cosmétique conforme à la présente invention peut également comprendre au moins un composé pâteux.

Par « pâteux » au sens de la présente invention, on entend un composé gras à changement d'état solide/liquide réversible et comportant à la température de 23 °C une fraction liquide et une fraction solide. On entend également par pâteux, le polylaurate de vinyle.

Un composé pâteux au sens de l'invention peut présenter avantageusement une dureté à 20 °C allant de 0,001 à 0,5 MPa, de préférence de 0,002 à 0,4 MPa.

Parmi les composés pâteux susceptibles d'être utilisés dans la composition selon l'invention, on peut citer les lanolines et les dérivés de lanoline comme les lanolines acétylées, les lanolines oxypropylénées ou le lanolate d'isopropyle, et leurs mélanges. On peut également utiliser des esters d'acides ou d'alcools gras, notamment ceux ayant 20 à 65 atomes de carbone comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux et leurs mélanges. Comme triglycéride d'origine végétale, on peut utiliser les dérivés d'huile de ricin hydrogénée, tels que le « THIXINR^{®} » de Rheox.

On peut également citer les polyesters résultant de l'estérification d'un acide carboxylique et d'un ester acide hydroxycarboxylique aliphatique. Par exemple, le RISOCAST^{®} DA-L (ester issu de la réaction d'estérification de l'huile de ricin hydrogéné avec de l'acide dilinoléïque dans des proportions de 2 pour 1) et le RISOCAST^{®} DA-H (ester résultant de l'estérification de l'huile de ricin hydrogénée avec de l'acide isostéarique dans des proportions de 4 pour 3) commercialisés par la société japonaise KOKYU ALCOHOL KOGYO.

Comme composé pâteux convenant avantageusement à la formulation des compositions cosmétiques conformes à la présente invention, on peut faire mention des coco-glycérides hydrogénés.

On peut aussi citer les composés pâteux siliconés tels que les polydiméthylsiloxanes (PDMS) de hauts poids moléculaires et en particulier ceux ayant des chaînes pendantes du type alkyle ou alcoxy ayant de 8 à 24 atomes de carbone, et un point de fusion de 20-55 °C, comme les stéaryl diméthicones notamment ceux vendus par la société DOW CORNING sous les noms commerciaux de DC2503^{®} et DC25514^{®} et leurs mélanges.

### MATIERE COLORANTE

Une composition cosmétique conforme à l'invention peut, en outre, comprendre au moins une matière colorante.

Selon un mode de réalisation, une ou des matières colorantes peuvent être ajoutées à une composition cosmétique selon l'invention de manière à ce que la transparence et/ou la translucidité de cette dernière n'en soit pas affectée.

Une telle matière colorante peut être par exemple choisie parmi les matières colorantes hydrosolubles ou non, liposolubles ou non, organiques ou inorganiques, notamment de type pigments ou nacres, classiquement utilisée dans les compositions cosmétiques, les matériaux à effet optique, et leurs mélanges.

Les matières colorantes peuvent être présentes à raison de 0,01 à 40 % en poids, notamment de 0,5 à 25 % en poids par rapport au poids total de la composition cosmétique.

Par pigments, il faut comprendre des particules blanches ou colorées, inorganiques (minérales) ou organiques, insolubles dans une solution aqueuse, destinées à colorer le film résultant.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome.

Il peut également s'agir de pigment ayant une structure qui peut être par exemple de type séricite/oxyde de fer brun/dioxyde de titane/silice. Un tel pigment est commercialisé par exemple sous la référence « COVERLEAF NS » ou « JS » par la société CHEMICALS AND CATALYSTS et présente un rapport de contraste voisin de 30.

La matière colorante peut encore comporter un pigment ayant une structure qui peut être par exemple de type microsphères de silice contenant de l'oxyde de fer. Un exemple de pigment présentant cette structure est celui commercialisé par la société MIYOSHI sous la référence « PC BALL PC-LL-100 P », ce pigment étant constitué de microsphères de silice contenant de l'oxyde de fer jaune.

Parmi les pigments organiques utilisables dans l'invention, on peut citer le noir de carbone, les pigments de type D & C, les laques à base de carmin de cochenille, de baryum, strontium, calcium, aluminium ou encore les dicétopyrrolopyrroles (DPP) décrits dans les documents EP-A-542669, EP-A-787730, EP-A-787731 et WO-A- 96/08537.

Par « nacres », il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica titane recouvert d' oxychlorure de bismuth, le mica titane recouvert avec de l'oxyde de chrome, le mica titane recouvert avec un colorant organique ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Parmi les nacres disponibles sur le marché, on peut citer les nacres « TIMICA », « FLAMENCO » et « DUOCHROME » (sur base de mica) commercialisées par la société ENGELHARD, les nacres « TIMIRON » commercialisées par la société MERCK, les nacres sur base de mica « PRESTIGE » commercialisées par la société ECKART et les nacres sur base de mica synthétique « SUNSHINE » commercialisées par la société SUN CHEMICAL.

Les nacres peuvent plus particulièrement posséder une couleur ou un reflet jaune, rose, rouge, bronze, orangé, brun, or et/ou cuivré.

A titre illustratif des nacres pouvant être mises en oeuvre dans le cadre de la présente invention, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté par exemple commercialisées par la société MERCK sous la dénomination Xirona Silver et les nacres orangées rosées vert doré notamment commercialisées par la société MERCK sous la dénomination Indian summer (Xirona) et leurs mélanges.

La composition cosmétique selon l'invention peut également contenir au moins un matériau à effet optique spécifique.

Cet effet est différent d'un simple effet de teinte conventionnel, c'est-à-dire unifié et stabilisé tel que produit par les matières colorantes classiques comme par exemple les pigments monochromatiques.

Au sens de l'invention, « stabilisé » signifie dénué d'effet de variabilité de la couleur avec l'angle d'observation ou encore en réponse à un changement de température.

Par exemple, ce matériau peut être choisi parmi les particules à reflet métallique, les agents de coloration goniochromatiques, les pigments diffractants, les agents thermochromes, les agents azurants optiques, ainsi que les fibres, notamment interférentielles. Bien entendu, ces différents matériaux peuvent être associés de manière à procurer la manifestation simultanée de deux effets, voire d'un nouvel effet conforme à l'invention.

Les particules à reflet métallique utilisables dans l'invention sont en particulier choisies parmi :
- les particules d'au moins un métal et/ou d'au moins un dérivé métallique,
- les particules comportant un substrat, organique ou minéral, monomatière ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple les bronzes et les laitons) sont des métaux préférés.

Par « dérivés métalliques », on désigne des composés dérivés de métaux notamment des oxydes, des fluorures, des chlorures et des sulfures

A titre illustratif de ces particules, on peut citer des particules d'aluminium, telles que celles commercialisées sous les dénominations « STARBRITE 1200 EAC^{®} » par la société SIBERLINE et « METALURE^{®} » par la société ECKART.

On peut également citer les poudres métalliques de cuivre ou des mélanges d'alliage telles les références 2844 commercialisées par la société RADIUM BRONZE, les pigments métalliques comme l'aluminium ou le bronze, telles que celles commercialisées sous les dénominations « ROTOSAFE 700 » de la société ECKART, les particules d'aluminium enrobé de silice commercialisées sous la dénomination « VISIONAIRE BRIGHT SILVER » de la société ECKART et les particules d'alliage métallique comme des poudres de bronze (alliage cuivre et zinc) enrobé de silice commercialisées sous la dénomination de « VISIONAIRE BRIGHT NATURAL GOLD » de la société ECKART.

Il peut encore s'agir de particules comportant un substrat de verre comme celles commercialisées par la société NIPPON SHEET GLASS sous les dénominations « MICROGLASS METASHINE ».

L'agent de coloration goniochromatique peut être choisi par exemple parmi les structures multicouches interférentielles et les agents de coloration à cristaux liquides.

Des exemples de structures multicouche interférentielles symétriques utilisables dans des compositions réalisées conformément à l'invention sont par exemple les structures suivantes : Al/SiO₂/Al/SiO₂/Al, des pigments ayant cette structure étant commercialisés par la société DUPONT DE NEMOURS ; Cr/MgF₂/Al/MgF₂/Cr, des pigments ayant cette structure étant commercialisés sous la dénomination « CHROMAFLAIR » par la société FLEX ; MoS₂/SiO₂/Al/SiO₂/MoS₂ ; Fe₂O₃/SiO₂/Al/SiO₂/Fe₂O₃, et Fe₂O₃/SiO₂/Fe₂O₃/SiO₂/Fe₂O₃, des pigments ayant ces structures étant commercialisés sous la dénomination « SICOPEARL » par la société BASF ; MoS₂/SiO₂/mica-oxyde/SiO₂/MoS₂ ; Fe₂O₃/SiO₂/mica-oxyde/SiO₂/Fe₂O₃ ; TiO₂/SiO₂/TiO₂ et TiO₂/Al₂O₃/TiO₂ ; SnO/TiO₂/SiO₂/TiO₂/SnO ; Fe₂O₃/SiO₂/Fe₂O3 ; SnO/mica/TiO₂/SiO₂/TiO₂/mica/SnO, des pigments ayant ces structures étant commercialisés sous la dénomination « XIRONA » par la société MERCK (Darmstadt). A titre d'exemple, ces pigments peuvent être les pigments de structure silice/oxyde de titane/oxyde d'étain commercialisés sous le nom « XIRONA MAGIC » par la société MERCK, les pigments de structure silice/oxyde de fer brun commercialisés sous le nom « XIRONA INDIAN SUMMER » par la société MERCK et les pigments de structure silice/oxyde de titane/mica/oxyde d'étain commercialisés sous le nom « XIRONA CARRIBEAN BLUE » par la société MERCK. On peut encore citer les pigments « INFINITE COLORS » de la société SHISEIDO. Selon l'épaisseur et la nature des différentes couches, on obtient différents effets. Ainsi, avec la structure Fe₂O₃/SiO₂/Al/ SiO₂/Fe₂O₃ on passe du doré-vert au gris-rouge pour des couches de SiO₂ de 320 à 350 nm ; du rouge au doré pour des couches de SiO₂ de 380 à 400 nm ; du violet au vert pour des couches de SiO₂ de 410 à 420 nm ; du cuivre au rouge pour des couches de SiO₂ de 430 à 440 nm.

On peut citer, à titre d'exemple de pigments à structure multicouche polymérique, ceux commercialisés par la société 3M sous la dénomination « COLOR GLITTER ».

Comme particules goniochromatiques à cristaux liquides, on peut utiliser par exemple celles vendues par la société CHENIX ainsi que celle commercialisées sous la dénomination « HELICONE^{®} HC » par la société WACKER.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine.

Les colorants hydrosolubles sont par exemple le jus de betterave, le bleu de méthylène.

Selon un mode de réalisation, une composition selon l'invention peut comprendre au moins une matière colorante par exemple choisie parmi les matières colorantes organiques et les matières colorantes inorganiques, telles que les pigments et les nacres, les matériaux à effet optique spécifique et leurs mélanges.

Selon un autre mode de réalisation, la composition selon l'invention est exempte de matière colorante.

### CHARGES

Les compositions cosmétiques conformes à l'invention peuvent également comprendre au moins une charge, de nature organique ou minérale.

Au sens de l'invention, les charges sont distinctes des matières colorantes.

Une ou des charges peuvent être avantageusement ajoutées à une composition cosmétique selon l'invention de telle sorte que ces propriétés, notamment de viscosité, de transparence, de translucidité, n'en soient pas substantiellement affectées.

Par « charge », il faut comprendre les particules incolores ou blanches, solides de toutes formes, qui se présentent sous une forme insoluble et dispersée dans le milieu de la composition. De nature minérale ou organique, elles permettent de conférer du corps ou de la rigidité à la composition, et/ou de la douceur, de la matité et de l'uniformité au maquillage. Elles sont distinctes des particules de silice pyrogénée.

Les charges utilisées dans les compositions selon la présente invention peuvent être de formes lamellaires, globulaires, sphériques, de fibres ou de toute autre forme intermédiaire entre ces formes définies.

Les charges selon l'invention peuvent être ou non enrobées superficiellement, et en particulier elles peuvent être traitées en surface par des silicones, des acides aminés, des dérivés fluorés ou toute autre substance favorisant la dispersion et la compatibilité de la charge dans la composition.

Parmi les charges minérales utilisables dans les compositions selon l'invention, on peut citer le talc, le mica, la silice, le siloxysilicate de triméthyle, le kaolin, la bentone, le carbonate de calcium précipité, le carbonate et l'hydrogéno-carbonate de magnésium, l'hydroxyapatite, le nitrure de bore, les microsphères de silice creuses (Silice Beads de Maprecos), les microcapsules de verre ou de céramique ; le Sunsphare L-31, le Sunphare H-31 commercialisés par Asahi Glass ; le Chemicelen commercialisé par Asahi Chemical ; les composites de silice et de dioxyde de titane comme la série TSG commercialisée par Nippon Sheet Glass, et leurs mélanges.

Parmi les charges organiques utilisables dans les compositions selon l'invention on peut citer les poudres de polyamide (Nylon^{®} Orgasol de chez Atochem), de poly-b-alanine et polyéthylène, les poudres de polytétrafluoroéthylène (Téflon®), la lauroyl-lysine, l'amidon, les poudres de polymères de tétrafluoroéthylène, les microsphères creuses de polymères telles l'EXPANCEL (NOBEL INDUSTRIE), le carbonate de calcium précipité, le carbonate et l'hydrogéno-carbonate de magnésium, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, le Polypore® L 200 (Chemdal Corporation), les microbilles de résine de silicone (Tospearl ® de Toshiba, par exemple), les poudres de poyuréthanne, en particulier les poudres de polyuréthanne réticulé comprenant un copolymère, ledit copolymère comprenant du triméthylol hexyllactone. En particulier, il peut s'agir d'un polymère d'hexaméthylène di-isocyanate/triméthylol hexyllactone. De telles particules sont notamment disponibles dans le commerce, par exemple sous la dénomination de PLASTIC POWDER D-400^{®} ou PLASTIC POWDER D-800^{®} de la société TOSHIKI, et leurs mélanges.

Une charge peut être présente dans une composition cosmétique conforme à l'invention à raison d'environ 0,1 à environ 30 % en poids de charge par rapport au poids total de la composition, de préférence d'environ 5 à environ 30 %.

Une charge convenant à l'invention peut être par exemple une charge dont la granulométrie moyenne est inférieure à 100 µm, notamment comprise entre 1 et 50 µm, par exemple entre 4 et 20 µm.

### ADDITIFS

Une composition cosmétique selon l'invention peut également comprendre en outre tout additif usuellement utilisé dans le domaine concerné, choisi parmi des agents filmogènes, et le cas échéant des auxiliaires de filmification, des gommes, des polymères semi-cristallins, des gélifiants, des agents émulsifiants et le cas échéant des agents co-émulsifiants, des agent antioxydants, des huiles essentielles, des conservateurs, des parfums, des actifs cosmétiques, des neutralisants, des agents hydratants, des agents antiseptiques, des vitamines telles que les vitamines B3 ou E et leurs dérivés, des agents protecteurs contre les UV, et leurs mélanges.

Comme actifs cosmétiques utilisables dans les compositions de l'invention, on peut citer les hydratants (polyol comme glycérine), les vitamines (C, A, E, F, B, ou PP), les acides gras essentiels, les huiles essentielles, les céramides, les sphingolipides, les filtres solaires liposolubles ou sous forme de nano-particules, les actifs spécifiques de traitement de la peau (agents de protection, anti-bactériens, anti-rides...).

Ces actifs peuvent être utilisés par exemple à des concentrations de 0 à 20 % et notamment de 0,001 à 15 % par rapport au poids total de la composition.

Il relève des opérations de routine de l'homme de l'art d'ajuster la nature et la quantité des additifs présents dans les compositions conformes à l'invention de telle sorte que les propriétés cosmétiques et les propriétés de viscosité désirées de ces dernières n'en soient pas affectées.

Selon un mode de réalisation, une composition conforme à l'invention peut comprendre à titre de polyoléfine de point de fusion inférieur ou égal à environ 40 °C, un polyisobutène hydrogénée, et à titre de particules de silice pyrogénée une silice pyrogénée hydrophobes, traitée en surface par du di-méthylsilane.

Selon une variante de réalisation, une composition selon l'invention peut, en outre, comprendre un polyamide de type condensat diacide en C36 hydrogène/éthylène diamine, estérifié par de l'alcool stéarylique, stabilisé (Uniclear 100 VG^{®}).

La présente invention a également pour objet une utilisation de particules de silice pyrogénée à titre d'agent de structuration d'une polyoléfine présentant un point de fusion inférieur ou égal à 40 °C pour la préparation d'une composition cosmétique.

Avantageusement, une telle composition peut présenter une dureté inférieure ou égale à 30 g, mesurée à environ 20 °C et ne s'écoule pas sous son propre poids.

Selon un mode de réalisation, la ou les polyoléfine(s) mise(s) en oeuvre dans l'utilisation conforme à l'invention peu(ven)t être telle(s) que définie(s) précédemment.

Selon un autre mode de réalisation, les particules de silice pyrogénée mises en oeuvre dans un mélange destiné à une utilisation conforme à l'invention peuvent être telles que définies précédemment.

Une composition selon l'invention peut se présenter sous une forme coulée, et par exemple, sous la forme d'un stick ou bâton, sous forme de pâte souple dans une bouillotte, ou sous la forme de coupelle.

Par exemple, elle peut constituer un fond de teint coulé, un fard à joues ou à paupières coulé, notamment coloré, un rouge à lèvres, un brillant pour les lèvres et notamment un gloss, un produit anti-cerne.

Une composition selon l'invention peut notamment se présenter sous la forme d'une composition de maquillage et/ou de soins des lèvres, en particulier un rouge à lèvres, un baume à lèvres, ou un gloss.

Comme indiqué précédemment, une composition cosmétique conforme à l'invention présente avantageusement des propriétés de transparence et de translucidité.

Au sens de l'invention cette propriété de transparence et de translucidité signifie qu'une couche de la composition d'une épaisseur donnée laisse passer une partie de la lumière visible.

Si cette partie de la lumière visible est diffusée, la composition sera définie comme étant une composition translucide, et si au contraire elle n'est pas diffusée, alors la composition sera définie comme étant une composition transparente.

La présente invention est illustrée à l'aide des exemples ci-après. Ces exemples ne constituent en aucun cas une limitation de la présente invention.

### Exemple 1

On prépare un produit de maquillage de type « gloss » pour les lèvres dont la composition est la suivante :

| Ingrédient | Quantité |
|---|---|
| Tri-méllitate de tri-décyle | 35,77 |
| Silice pyrogénée hydrophobe, traitée en surface par di-méthylsilane (AEROSIL R 972 de Degussa) | 8 |
| Polyisobutène hydrogéné (PARLEAM de NOF) | 35,77 |
| Polybutène (INDOPOL H 100) | 20 |
| Conservateurs | Qs |
| Total | 100 |

Le mode opératoire utilisé est le suivant. Le polybutène, le polyisobutylène, le tri-méllitate de tri-décyle et les conservateurs, sont mélangés à une température d'environ 90-100 °C.

Une fois le mélange homogène, on ajoute progressivement le parfum, et les particules de silice pyrogénée en maintenant la température à 100-105 °C au Rayneri.

Le mélange est agité jusqu'à homogénéisation complète et obtention d'un mélange translucide.

Pour plus de transparence, on peut soumettre ce mélange au Rayneri sous-vide pour éliminer les éventuelles bulles d'air présentes dans le produit fini.

La composition est ensuite coulée dans un moule.

### Exemple 2

On prépare un produit de maquillage de type « gloss » pour les lèvres dont la composition est la suivante :

| Ingrédient | Quantité |
|---|---|
| Tri-méllitate de tri-décyle | 34,07 |
| Polyisobutène hydrogéné (PARLEAM de NOF) | 34,07 |
| Polybutène (INDOPOL H 100) | 20 |
| Condensat diacide en C₃₆ hydrogéné/éthylène diamine, estérifié par alcool stéarylique (poids moléculaire d'environ 4 000) stabilisé (Uniclear 100 VG^{®} de ARIZONA CHEMICAL) | 0,4 |
| Silice pyrogénée hydrophobe, traitée en surface par di-méthylsilane (AEROSIL R 972 de Degussa) | 10,5 |
| Conservateurs | qs |
| Parfum | 0,5 |
| Total | 100 |

Le mode opératoire utilisé est le suivant. Le polybutène, le polyisobutylène, le tri-méllitate de tri-décyle, le polyamide et, les conservateurs, sont mélangés à température de fusion du polyamide, soit environ 105 °C.

Une fois le mélange homogène et le polyamide bien fondu, on ajoute progressivement le parfum, et les particules de silice pyrogénée en maintenant la température à 100-105 °C au Rayneri.

Le mélange est agité jusqu'à homogénéisation complète et obtention d'un mélange translucide.

Pour plus de transparence, on peut soumettre ce mélange au Rayneri sous-vide pour éliminer les éventuelles bulles d'air présentes dans le produit fini.

La composition est ensuite coulée dans un moule.

## Revendications

1. Composition cosmétique, de maquillage et/ou de soin des matières kératiniques, comprenant au moins une polyoléfine présentant un point de fusion inférieur ou égal à environ 40 °C et des particules de silice pyrogénée, ladite composition ne s'écoulant pas sous son propre poids et présentant une dureté inférieure ou égale à environ 30 g, mesurée à environ 20 °C.

2. Composition selon la revendication précédente, dans laquelle la dureté varie d'environ 6 g à environ 30 g, en particulier d'environ 10 g à environ 25 g, et plus particulièrement étant d'environ 15 g, mesurée à environ 20 °C.

3. Composition selon la revendication 1 ou 2, dans laquelle ladite polyoléfine est liquide à température ambiante (20-25 °C) et à pression atmosphérique.

4. Composition selon l'une quelconque des revendications précédentes, dans laquelle la polyoléfine présente une masse molaire variant d'environ 400 à environ 10 000 g/mol, en particulier d'environ 600 à environ 7 500 g/mol et plus particulièrement variant d'environ 650 à environ 5000 g/mol.

5. Composition selon l'une quelconque des revendications précédentes, dans laquelle la polyoléfine est hydrogénée.

6. Composition selon l'une quelconque des revendications précédentes, dans laquelle la polyoléfine est une polyalphaoléfine.

7. Composition selon l'une quelconque des revendications précédentes, dans laquelle la polyoléfine est choisie parmi des polybutylènes, des polyisobutylènes hydrogénés ou non, et des polydécènes hydrogénés ou non, et leurs mélanges.

8. Composition selon l'une quelconque des revendications précédentes, dans laquelle la polyoléfine est présente en une teneur variant d'environ 5 à environ 90 % en poids, en particulier d'environ 10 à environ 60 % en poids, et plus particulièrement d'environ 30 % à environ 40 % en poids par rapport au poids total de la composition.

9. Composition selon l'une quelconque des revendications précédentes, dans laquelle les particules de silice pyrogénée sont choisies parmi des particules de silice pyrogénée hydrophile et des particules de silice pyrogénée hydrophobe.

10. Composition selon l'une quelconque des revendications précédentes, dans laquelle les particules de silice pyrogénée sont présentes en une teneur variant d'environ à 1 % à environ 30 % en poids, en particulier d'environ 5 % à environ 20 % en poids, et plus particulièrement d'environ 8 % à environ 15 % en poids par rapport au poids total de la composition.

11. Composition selon l'une quelconque des revendications précédentes, comprenant en outre au moins un polyamide de masse moléculaire moyenne inférieure à 100 000 comportant a) un squelette polymérique ayant des motifs de répétition hydrocarbonés pourvus d'au moins une fonction amide, b) optionnellement une chaîne grasse pendante et/ou au moins une chaîne grasse terminale, éventuellement fonctionnalisée, ayant de 6 à 120 atomes de carbones, et étant liée à ces motifs hydrocarbonés.

12. Composition selon la revendication précédente, dans laquelle les chaînes grasses représentent de 40 à 98 % du nombre total des motifs amide et des chaînes grasses, en particulier de 50 à 95 % du nombre total des motifs amide et des chaînes grasses.

13. Composition selon la revendication 11 ou 12, dans laquelle la masse moléculaire moyenne du polyamide varie de 1000 à 100 000, en particulier de 1000 à 50 000, en particulier de 1 000 à 30 000, en particulier de 2 000 à 20 000, et plus particulièrement de 2 000 à 10 000 g/mol.

14. Composition selon l'une quelconque des revendications 11 à 13, dans laquelle la ou les chaînes grasses terminales sont liées au squelette par des groupes ester.

15. Composition selon l'une quelconque des revendications 11 à 14, dans laquelle la ou les chaînes grasses ont de 12 à 68 atomes de carbone.

16. Composition selon l'une quelconque des revendications 11 à 15, dans laquelle le polyamide est formule générale (I) suivante : 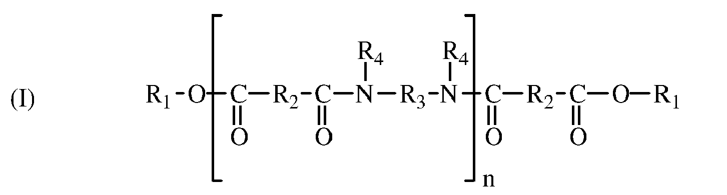 dans laquelle :
- n désigne un nombre entier de motifs amide tel que le nombre de groupes ester représente de 10 % à 50 % du nombre total des groupes ester et amide ;
- chacun des symboles R₁ désigne indépendamment un groupe alkyle ou alcényle ayant au moins 4 atomes de carbone, et en particulier de 4 à 24 atomes de carbone ;
- chacun des symboles R₂ représente indépendamment un groupe hydrocarboné en C4 à C42 à condition que 50 % des groupes R₂ représentent un groupe hydrocarboné en C30 à C42 ;
- chacun des symboles R₃ représente indépendamment un groupe organique pourvu d'au moins 2 atomes de carbone, d'atomes d'hydrogène et optionnellement d'un ou plusieurs atomes d'oxygène ou d'azote ;
- et chacun des symboles R₄ représente indépendamment un atome d'hydrogène, un groupe alkyle en C1 à C10 ou une liaison directe à R₃ ou à un autre R₄ de sorte que l'atome d'azote auquel sont liés à la fois R₃ et R₄ fasse partie d'une structure hétérocyclique définie par R4-N-R3, avec au moins 50 % des R₄ représentant un atome d'hydrogène.

17. Composition selon la revendication précédente, dans laquelle R₁ est un groupe alkyle en C₁₂ à C₂₂.

18. Composition selon la revendications 16 ou 17, dans laquelle 50 % des R₂ sont des groupes hydrocarbonés ayant de 30 à 42 atomes de carbone.

19. Composition selon l'une quelconque des revendications 11 à 18, dans laquelle le polyamide est choisi parmi les copolymères de diacide en C₃₆ condensés sur une éthylène diamine de masse moléculaire en poids d'environ 6 000, et leurs mélanges.

20. Composition selon l'une quelconque des revendications 11 à 19, dans laquelle le polyamide est présent en une teneur variant d'environ 0,01 à environ 20 % en poids, en particulier d'environ 0,1 à environ 10 % en poids, et plus particulièrement d'environ 0,2 % à environ 4 % en poids par rapport au poids total de la composition.

21. Composition selon l'une quelconque des revendications précédentes, comprenant en outre au moins une phase grasse additionnelle choisie parmi une phase grasse liquide, une phase grasse solide, et leur mélange.

22. Composition selon l'une quelconque des revendications précédentes, ladite composition comprenant une teneur en cire inférieure ou égale à 5 % en poids par rapport au poids total de la composition.

23. Composition selon l'une quelconque des revendications précédentes, comprenant en outre au moins une matière colorante.

24. Composition selon l'une quelconque des revendications précédentes, ladite composition étant anhydre.

25. Composition selon l'une quelconque des revendications précédentes, ladite composition étant une composition de maquillage et/ou de soin des lèvres.

26. Composition selon l'une quelconque des revendications précédentes, ladite composition se présentant sous la forme d'un gloss.

27. Composition selon l'une quelconque des revendications 1 à 25, ladite composition se présentant sous une forme compacte.

28. Utilisation de particules de silice pyrogénées à titre d'agent de structuration d'une polyoléfine présentant un point de fusion inférieur ou égal à 40 °C pour la préparation d'une composition cosmétique.

29. Utilisation selon la revendication précédente, dans laquelle ladite polyoléfine est telle que définie selon l'une quelconque des revendications 3 à 7.

30. Utilisation selon la revendication 28 ou 29, dans laquelle les particules de silice pyrogénée sont telles que définies selon la revendication 9.

31. Procédé de maquillage et/ou de soin de matières kératiniques comprenant au moins une étape consistant à appliquer sur au moins une partie desdites matières kératiniques une composition telle que définie selon l'une quelconque des revendications 1 à 27.
